# EUROPEAN PATENT APPLICATION

(11) **EP 1 710 325 A1**
(43) Date of publication of application: **11.10.2006**
(21) Application number: 03772352.5
(22) Date of filing: 13.11.2003
(51) Int. Cl.: C23C 14/58, C23C 14/48, C23C 14/18, C23C 14/24, A61C 8/00, A61F 2/30

(54) **METHOD OF PRODUCING ENDOSSEOUS IMPLANTS OR MEDICAL PROSTHESES BY MEANS OF IONIC IMPLANTATION, AND ENDOSSEOUS IMPLANT OR MEDICAL PROSTHESIS THUS PRODUCED**

(71) Applicant: Lifenova Biomedical, S.A., 20009 San Sebastian (Guipuzcoa) (ES)
(72) Inventor: ONATE DELA PRESA, José, Ignacio, 20009 San Sebastian (Guipuzcoa) (ES); ALAVA MARQUINEZ, Jose, Inaki, 20009 San Sebastian (Guipuzcoa) (ES); DE MAEZTU MARTINEZ, Miguel, Angel, 20009 San Sebastian (Guipuzcoa) (ES); BRACERAS IZAGUIRRE, Inigo, 20009 San Sebastian (Guipuzcoa) (ES); GARCIA LUIS, Alberto, 20009 San Sebastian (Guipuzcoa) (ES)
(74) Representative: Carpintero Lopez, Francisco
(86) International application number: PCT/ES2003/000575
(87) International publication number: WO 2005/047559

(57) **Abstract**

This refers to a method to obtain medical implants or prostheses with a surface microrugosity and/or an induced oxide layer, treated by ion implantation with controlled amounts of elements such as CO, C, H, N or O in endo-osseous implants or prostheses made from metals, metal alloys or biocompatible composite materials. This surface treatment produces some modifications in the characteristics of the surface of the endo-osseous implants or medical prostheses in relation to their nanorugosity, hydrophilic properties and chemical composition that significantly increases the degree of osseointegration thereof.

## Description

### OBJECT OF THE INVENTION

The method of the invention can be applied to obtain endosseous implants or medical prostheses treated with ion implantation to achieve good osseointegration properties.

An object of this invention corresponds to a method to obtain the prosthesis which, as well as ion implantation, also produces an induced microrugosity in the implant and an induced oxidation on parts in contact with the bone, obtaining improved properties of osseointegration and reduced ion implantation treatment times, resulting in a reduction in costs for the process as a whole.

Another object of this invention is an implant obtained according to the method of the invention that presents good osseointegration properties.

### BACKGROUND OF THE INVENTION

There is a growing need for prosthetic treatments of long duration (implants or prostheses of the knee, hip, maxillofacial, cranial etc) in daily clinical practice. This has led to the use, in many cases, of metallic materials (subcutaneous or osseous implants) especially in patients subjected major surgery, maxillofacial surgery and in osteoporotic and osteoproliferative patients. However, these systems of prosthetic replacement have an important failure rate, over 30% in some cases, sometimes making it impossible to recur to this technique.

The most frequent complications described in the medical literature, are the infectious type (infection of the implant, bacterium, sepsis, and other rarer complications, such as gangrene, etc.), inflammatory type (reaction to foreign body, local inflammation, total rejection), problems associated with tissue integration (gingivitis, sinovial metallosis, osteoresorption) and those arising from their handling and use (bone fractures, failed metal-tissue interface).

These complications are commonly associated with biocompatibility, which explains why biocompatible metals constitute the most important and diverse group of materials used in biomedical applications, owing to their good biocompatibility properties and chemical inertia, making them suitable for contact with tissues and biological fluids. Another important trait is that they can be manufactured in a wide range of forms.

However, the advances made in recent years in relation to the types of alloys used have not reduced the amount of complications as much as expected, and the experimental procedures used to improve their biocompatibility have been restricted to more permeable designs or superficial impregnations, more or less intense, with biologically active molecules (antibiotics, antiseptics, anti-aggregants, etc.).

There is, therefore, a need to develop medical materials which can be used to solve some, or all, of the abovementioned complications, which would also result in shorter treatment times for patients.

On the other hand, overcoming these problems is only the beginning, since, once these have been solved, there remains the problem of prolonged treatment times for patients diagnosed as requiring a prosthesis or implant. This is largely due to the time required for the implant or prosthesis to integrate with the surrounding osseous tissue, in other words, the time required for a good osseointegration. Osseointegration conditions the operativity of the implants and prostheses and is usually only achieved several months after the surgical operation.

In this context, osseointegration of implants and prostheses faces two important challenges:
- To accelerate osseointegration (percentage of surface area of the implant or prosthesis in contact with the surrounding bone).
- To achieve good levels of osseointegration in zones of poor bone density.

In that concerning the problem of osseointegration of the prostheses or implants, a method of approaching the problem could consist in applying a surface treatment thereon which confers upon them the appropriate characteristics.

This is the case of ion implantation, a treatment which does not modify the structural properties or the dimensional tolerances of the treated prostheses or implants but which, however, can modify their surface properties by means of the introduction of a series of selected ions on the surface, modifying the properties thereof in the desired sense.

Use has been made of different techniques of ion implantation for many years in different fields of application with the object of modifying the surface properties of the components. It is used, for example, in electronics for modification of the electrical properties of semiconductors. It is also applied in the metal mechanics industry for the improvement of properties of resistance to wear and corrosion, in cases such as moulds and injection mouthpieces, machining and cutting tools, gauges, etc.

Ion implantation has also been used on biomaterials. This is the case, for example, of the implantation of germicidal elements in medical equipment described in U.S. Pat. No. 5492763, or the implantation in implants of cobalt-chromium alloys with the object of increasing surface hardness and reducing friction as described in European patent 0 526 581. There are also patents which improve the tribological properties of metallic materials and, for example, in the following Patents: WO91/16013, in which resistance to abrasion is increased, resisting the fretting wear by achieving reduced friction, US 4568396, which reduces the wear and increases resistance to fatigue by fretting and GB 2154450, which achieves a hardening.

Ion implantation is also used for polymeric materials such as in Patents GB 2286347, WO01/49339, which improve wearing properties and compatibility, Patent FR8806890, which describes a reduction in the wear and rubbing coefficient and wear, or in Patent US 5133757, according to which implantation is applied to the surface of the prosthetic components and implants that, when functioning, move relative to each other. In Patents US 6217615 and US 6051751, adhesion of cements in the prostheses is improved. Patent US 4693760 tackles prevention of the discoloration of orthopedic implants by ion implantation treatment.

The problem of osseointegration has also been broached from the ion implantation technique in order to produce a surface coated with hydroxyapatite, a coating which has also been applied by other processes. Such is the case of the method for the production of surgical implantations coated with synthetic bone described in Spanish patent ES 2.006.658 which employs high energy beams of xenon to coat the implants with hydroxyapatite by the sputtering technique or cathodic spraying. German patent application DE 19830530 describes the production of titanium surfaces coated with calcium phosphate by ion implantation. In this last case, use is made of phosphorus and calcium implantation followed by a heat treatment.

The applicant is the holder of the patent application PCT WO02/083977, which describes a method to manufacture endo-osseous implants or prostheses from a base material that is subjected to a surface treatment of ion implantation of, at least, one element selected from C, O, H, Xe, Ar, He, Kr, Ne and/or a compound comprising one or more of these elements, obtaining improvements in the degree of osseointegration of the implant and/or a degree of ionic lixivation to the physiologic medium in contact with the implant and/or improved tribological properties.

### DESCRIPTIÓN OF THE INVENTIÓN

The object of the invention is to solve the problem described here, specifically, to obtain endo-osseous implants and prostheses, superficially treated by ion implantation, which present improved characteristics of osseointegration.

The present invention refers to a method to obtain implants or medical prostheses, designed to improve their properties of osseointegration in osseous structures, based on that the implant or prosthesis presents an induced microrugosity, at least in areas intended to be in contact with the bone, and/or an induced oxide layer, at least on the surfaces intended to be in contact with the bone, and a surface subjected to ion implantation treatment of controlled quantities of certain elements and/or compounds, at least in areas intended to be in contact with the bone.

The method of the invention comprises the following steps:
a) Manufacturing the endo-osseous implant or medical prosthesis from a metal alloy or metal matrix composite.
b) Producing a microrugosity on the endo-ossous implant or medical prosthesis, at least on the surface intended to be in contact with the bone.
c) Creating or growing an oxide layer on the endosseous implant or medical prosthesis, at least on the surface intended to be in contact with the bone,
d) Subjecting the endosseous implant or medical prosthesis to an ion implantation treatment, at least the surface intended to be in contact with the bone, with at least one of the ions C, O, H, N, CO and/or a compound comprising one or several of the said ions.

Steps b) and c) can be optional, as the process can incorporate steps a), b) and d), steps a), c) and d) or steps a), b), c) and d), and steps can be carried out in a different order to that specified here, depending on the characteristics of the implant required.

Specifically, endo-osseous implant and/or medical prostheses are obtained with an enhanced degree of osseointegration, and/or a reduced degree of ionic lixiviation to the physiologic medium.

Moreover, with the method of the invention, the ion implantation treatment is less costly, in terms of reduced treatment times, owing to complementation with a surface microrugosity and/or with an induced oxide layer.

### DESCRIPTION OF THE DIAGRAMS

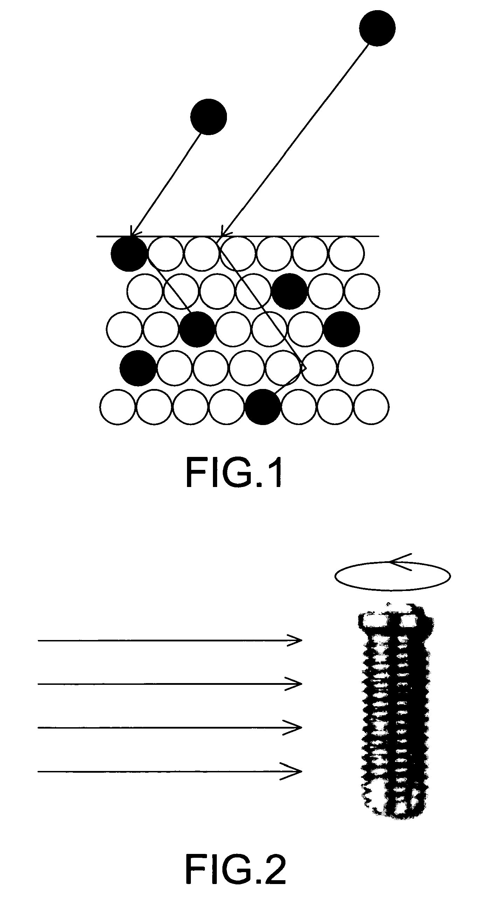

In FIG. 1 a simplified diagram of the ion implantation process can be seen, in which the ions are accelerated by application of high electromagnetic fields, and impact on the surface of the material, being inserted in the material. This process is carried out without originating any modification in the surface dimensions of the implanted material, but nevertheless its physico-chemical-topographic properties are modified.
In FIG. 2 detail of an embodiment is shown in which the beam of ions impacts directly on a dental implant, at the same time as the latter is subjected to a rotational movement. The beam can impact the piece from different directions, so that it is assured that the whole surface of the implant is subjected to the ion implantation treatment.
In FIG. 3 a simplified schematic of a typical process for manufacturing dental implants can be seen.
In FIG. 4 the surface composition of a titanium Ti6A14V alloy analyzed by XPS (X-ray Photoelectron Spectroscopy) after anodizing and ion implantation, according to the process of the invention is shown.

### PREFERRED EMBODIMENT OF THE INVENTION

The invention refers to endo-osseous implants or medical prostheses, being manufactured from a base material that presents, at least on the surface intended to be in contact with the bone tissue, an induced microrugosity and/or an induced oxide layer growing, at least on the surface intended to be in contact with the tissue, the surface of which has been treated with ion implantation with, at least, one ion selected from among the ions C, O, H, N, CO and/or a compound that comprises one or more of these ions, in which ion beam energy between 0.2 keV and 1 MeV is applied, in which the ionic implantation process is carried out in a vacuum chamber at a pressure higher than 1 millibar and a dose of, at least, 10¹⁵ ions/ cm² is applied.

The invention also refers to a method by which implants and medical prostheses can be obtained with characteristics of enhanced osseointegration that comprises the following steps:
a) Manufacturing an endo-osseous implant or medical prosthesis from a metal alloy or metallic matrix composite.
b) Producing a microrugosity on the implant, at least on the surface intended to be in contact with the osseous tissue.
c) Growing an oxide layer on the implant, at least on the surface intended to be in contact with the osseous tissue.
d) Subjecting the endosseous implant or medical prosthesis to a surface ion implantation treatment with, at least, one element selected from among the ions C, O, H, N, CO and/or a compound that comprises one or more of these ions, in which an ion beam energy is used ranging from 0.2 keV to 1 MeV, in which ion implantation is carried out in a vacuum chamber with a pressure higher than 1 millibar applied at a dose of, at least, 10¹⁵ ions/cm².

The method comprises the previously mentioned steps, carried out in any order including, at least, the induced microrugosity step or the oxide layer growing step and the ion implantation treatment.

More specifically, the method can include all the steps described a), b), c) and d), or only steps a), b) and d), or steps a), c) and d).

Moreover, these steps can be carried out in a different order to the one described, for example in the following orders: a), b), c) and d); a), c), b) and d); a), b), d) and c); a), d), c) and b); a), d), b) and c) or a), c), d) and b).

The term endo-osseous implants or medical prostheses, as it is employed in this description includes whatever endo-osseous implant or prostheses intended to be in contact with living tissues or cells, or with corporal or biological fluids.

With regard to the base material any metal, metallic alloy, biocompatible material, and mixtures or composites thereof can be used in the elaboration of endo-osseous implants and/or medical prostheses, such as those materials which satisfy the standard UNE-EN ISO 10993. In a particular embodiment, said base material is selected among titanium; alloys of titanium, aluminium and vanadium, for example, Ti-6Al-4V; alloys of chromium and cobalt (Cr--Co); alloys of cobalt, chromium and molybdenum (Co--Cr--Mo), stainless steel, for example, AISI 316 stainless steel, etc.

According to the method of the invention, the microrugosity produced, at least on the surface intended to be in contact with bone, is produced by micro-shot-peening or shot-blasting and has a value ranging from 0.5 to 10 µm Ra.

The oxide layer induced, on at least the surface intended to be in contact with the bone, is produced by chemical attack, anodizing, heat treatment, acid attack at temperature or chemical conversion, and this typically has a thickness greater than 15 nanometres.

The method of the invention comprises the implantation of, at least, one ion of an element selected from among the ions C, O, H, N, CO and/or of an ion of a compound that comprises one or more of these ions, for example, CO, COn, CxHy, etc. (where n is a whole number between 1 and 3, and x and y are whole numbers between 1 and 100.)

The method of the invention is, preferably, carried out in a vacuum chamber with a vacuum of, at least, 1 millibar.

Ion implantation, according to the method of the invention, can be carried out, optionally, in presence of a residual atmosphere in said vacuum chamber. This residual atmosphere can consist both in the presence of oxygen and of residual organic compounds, for example, organic compounds produced by the evaporation of an organic compound during the process of ion implantation in the treatment chamber. The implanted ionic doses can vary within a wide range depending on the nature of the implanted ion, being, in general, greater than 10¹⁵ ions/cm² with the object of providing the endo-osseous implant or the medical prostheses with the necessary properties to achieve a significant enhancement of the osseointegration capacity.

The process of ion implantation according to the method of the invention can be carried out over a wide temperature range, for example, it can be carried out at a temperature between -120°C and 800°C, preferably, between ambient temperature and 250°C. In a particular embodiment, with the object of favouring mechanisms for diffusion, precipitation or transformation of compounds, the process of ion implantation according to the method of the invention can be carried out at a temperature of between 250°C. and 800°C. In other applications, these same mechanisms for diffusion, precipitation or transformation can be achieved by means of heat treatment of the endo-osseous implants or prostheses, when the process of ion implantation has been completed, at a temperature of between 250°C. and 800°C.

The ion implantation treatment, according to the method of the invention, can be applied to endo-osseous implants or medical prostheses by means of techniques of line of sight ion implantation, plasma immersion ion implantation or by means of whatever other equivalent technique of ionic bombardment.

Ion implantation, according to the method of the invention, produces a nanotextured surface (of approximately 3 to 6 nm Ra on a previously mirror polished surface), on the microrugose surface, providing more anchor points for the cells and, therefore, an enhanced osseointegration.

The method of the invention produces a surface rich in carbon in which the composition of the oxide layer contains an average of more than 20% of carbon in at least the first 20 nanometres of thickness.

The carbon surface has graphitic bonds, titanium carbides rich in carbon, titanium carbides or CO bonds.

More specifically, the presence of more than 10% of graphitic bonds along, at least, the first 10 nanometres of thickness is obtained.

As a result of the method of the invention endo-osseous implants or medical prostheses can be obtained, for example, dental implants, prostheses of hip, knee, etc., with an enhanced degree of osseointegration thereof, and/or with a reduced degree of lixiviation of ions to the physiological medium in contact with said implants and/or prostheses.

Below, some examples of implants according to the object of the invention are described.

### EXAMPLE 1

Ion implantation of CO+ ions is applied to a titanium dental implant with an induced titanium oxide layer of approximately 50nm.

This example illustrates the application of a surface ion implantation treatment of CO⁺ ions in a dental implant manufactured in titanium.

This corresponds to screws with an induced titanium oxide layer of approximately 50nm manufactured in alloy Ti6A14V.

Dental implants were subjected to anodizing treatment producing a layer of titanium oxide of approximately 50 nanometres.

Afterwards, the dental implants were cleaned successively in an ultrasonic bath of acetone and ethanol for a minimum period of time of 5 minutes. Subsequently they were all introduced in the vacuum chamber. The vacuum level that was reached and maintained during the entire ion implantation process was at all times higher than 5.10⁻⁷ millibars.

The ion implantation treatment was carried out in an Ion Implanter of the 1090 series by Danfysik AS. The dental implants were implanted ionically with CO+ ions, at an energy of 30 keV with a dose of 6.10¹⁷ ions/cm². Treatment was applied to the lateral cylindrical surface and the end surface of the thread of the dental implants.The temperature of the dental implants did not exceed 170°C at any time.

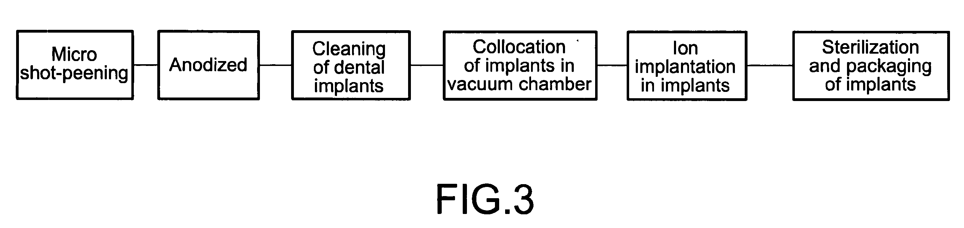

In figure 3, a simplified schematic of a typical manufacturing process of dental implants can be observed.

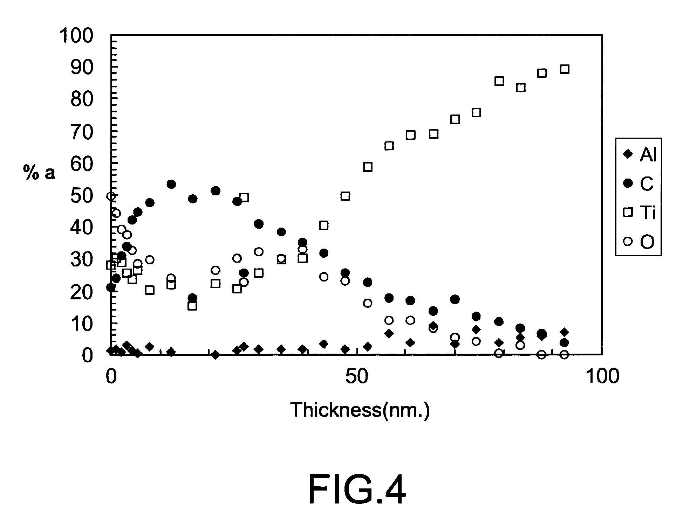

Figure 4 shows the chemical composition of the resulting surface where the carbon composition at some points greatly exceeds 50%. The composition was analyzed using the XPS technique (X-ray Photoelectron Spectroscopy).

The chemical composition obtained is directly related to the chemical compositions obtained in non-anodized samples but with more prolonged ion implantation treatments. These treatments, in turn, have been previously related to good properties of osseointegration at the surface, such as those described in patent application PCT ES02/00178.

### EXAMPLE 2

Ion implantation of C⁺ ions is produced in a titanium dental implant with an induced titanium oxide layer of approximately 50nm on a microrugosity of approximately 2µm Ra.

This example illustrates the application of a surface ion implantation treatment of CO⁺ ions in a dental implant manufactured in titanium.

This corresponds to screws with an induced titanium oxide layer of approximately 50nm on a microrugosity of approximately 2µm Ra manufactured in alloy Ti6A14V.

The dental implants were subjected to micro-shot-peening, obtaining a surface rugosity of 2µm Ra and, later, to an anodizing treatment producing a layer of titanium oxide of approximately 50 nanometres. Afterwards, the dental implants were cleaned successively in an ultrasonic bath of acetone and ethanol for a minimum period of time of 5 minutes. Subsequently they were all introduced in the vacuum chamber. The vacuum level that was reached and maintained during the entire ion implantation process was at all times higher than 5.10⁻⁷ millibars.

The ion implantation treatment was carried out in an Ion Implanter of the 1090 series by Danfysik AS. The dental implants were implanted ionically with CO+ ions, at an energy of 20 keV with a dose of 6.10¹⁷ ions/cm². Treatment was applied to the lateral cylindrical surface and the end surface of the thread of the dental implants.

The temperature of the dental implants did not exceed 170°C at any time.

In figure 3 a simplified schematic of a typical manufacturing process of dental implants can be observed.

## Claims

1. Method for the production of endosseous implants or medical prostheses by ion implantation that comprises:
a) Manufacturing the endosseous implant or medical prostheses from a metal alloy or metallic matrix composite.
b) Subjecting the endosseous implant or medical prosthesis to an ion implantation treatment, at least on the surface intended to be in contact with the bone with, at least, one of the following ions C, O, H, N, CO and/or a compound that comprises one or more of the said ions.
and, at least, one of the following steps:
c) Producing a microrugosity on the endo-osseous implant or medical prosthesis, at least on the surface intended to be in contact with the bone.
d) Creating or growing an oxide layer on the endo-osseous implant or medical prosthesis, at least on the surface intended to be in contact with the bone,
wherein steps b), c) and d) can be carried out in any order, according to the characteristics of the implant and the procedure followed.

2. Method for the production of endo-osseous implants or medical prostheses by ion implantation, according to claim 1, that comprises steps a) b) and c).

3. Method for the production of endo-osseous implants or medical prostheses by ion implantation, according to claim 1, that comprises steps a), b) and d).

4. Method for the production of endo-osseous implants or medical prostheses by ion implantation, according to claim 1, that comprises phases a), b), c) and d).

5. Method for the production of endo-osseous implants or medical prostheses by ion implantation, according to claims 1, 2 and 4, **characterized in that** the microrugosity produced, at least, on the surface intended to be in contact with the bone, is produced by micro-shot-peening or shot-blasting.

6. Method for the production of endo-osseous implants or medical prostheses by ion implantation, according to claims 1, 2 and 4, **characterized in that** the microrugosity has a typical value of between 0.5 and 10 µm Ra.

7. Method for the production of endo-osseous implants or medical prostheses by ion implantation, according to claims 1, 3 and 4, **characterized in that** the induced oxide layer, at least on the surface intended to be in contact with the bone, is produced by chemical attack, anodizing, heat treatment, acid attack at temperature or chemical conversion.

8. Method for the production of endo-osseous implants or medical prostheses by ion implantation, according to claims 1, 3 and 4, **characterized in that** the oxide layer has a thickness higher than 15 nanometres.

9. Method for the production of endo-osseous implants or medical prostheses by ion implantation, according to claims 1, 2, 3 and 4 in which the ion implantation treatment of, at least, the surface intended to be in contact with the bone, produces a carbon-rich surface for which the composition of the surface oxide layer contains an average of more than 20% carbon in at least the first 20 nanometres of thickness.

10. Method for the production of endo-osseous implants or medical prostheses by ion implantation, according to claims 1, 2, 3 and 4, **characterized in that** the ion implantation treatment of, at least, the surface intended to be in contact with the bone, produces a carbon-rich surface which is **characterized by** the presence of graphite bonds, titanium carbides rich in carbon and titanium carbides.

11. Method for the production of endo-osseous implants or medical prostheses by ion implantation, according to claims 1, 2, 3 and 4, **characterized in that** the ion implantation treatment, at least at the surface intended to be in contact with the bone, produces a carbon-rich surface that is **characterized by** the presence of graphite bonds, carbon-rich titanium carbides, titanium carbides and CO bonds.

12. Method for the production of endo-osseous implants or medical prostheses by ion implantation according to claims 1, 2, 3 and 4, **characterized in that** the ion implantation treatment, at least at the surface intended to be in contact with the bone, produces a carbon-rich surface **characterized by** the presence of more than 10% graphite bonds along at least the first 10 nanometres of thickness.

13. Method for the production of endo-osseous implants or medical prostheses by ion implantation, according to claims 1, 2, 3 and 4, **characterized in that** the implant obtained in phase a) has been made from titanium, titanium alloys, cobalt-chromium base alloys, stainless steel or a metallic matrix composite with some of these alloys.

14. Method for the production of endo-osseous implants or medical prostheses by ion implantation, according to claims 1, 2, 3 and 4, **characterized in that** the ion implantation process of step b) produces a nanotextured surface.

15. Method for the production of endo-osseous implants or medical prostheses by ion implantation, according to claim 14, **characterized in that** the nanotextured surface presents a rugosity of approximately 3 to 6 nm Ra on a previously mirror polished surface.

16. Method for the production of endo-osseous implants or medical prostheses by ion implantation according to claims 1, 2 3 and 4, **characterized in that** the ion implantation of phase b) produces a surface with better hydrophilic properties than the initial surface.

17. Method for the production of endo-osseous implants or medical prostheses by ion implantation, according to claims 1, 2 3 and 4, **characterized in that** the ion implantation of phase b) takes place in an atmosphere of residual oxygen, CO2 or some organic compound in the treatment chamber.

18. Method for the production of endo-osseous implants or medical prostheses by ion implantation, according to claims 1, 2 3 and 4, **characterized in that** the ion implantation treatment of phase b) takes place during the evaporation of an organic compound in the treatment chamber.

19. Method for the production of endo-osseous implants or medical prostheses by ion implantation according to claims 1, 2 3 and 4, **characterized in that** the ion implantation treatment of phase b) is applied by the technique of "line of sight ion implantation" or "beam ion implantation", "plasma immersion ion implantation" or "plasma source ion implantation", or a technique of ionic bombardment.

20. Method for the production of endo-osseous implants or medical prostheses by ion implantation, according to claims 1, 2 3 and 4, **characterized in that** the process of ion implantation described in phase b) is applied using an energy of 200eV to 500 KeV, in a treatment chamber with a better vacuum than 1 millibar and an implanted dose of, at least, 10¹⁵ ions/cm².

21. Method for the production of endo-osseous implants or medical prostheses by ion implantation, according to claims 1, 2 3 and 4, **characterized in that** the ion implantation process described in phase b) is applied at a temperature ranging from -120° C to 800° C.

22. Method for the production of endo-osseous implants or medical prostheses by ion implantation, according to claims 1, 2 3 and 4, **characterized in that**, after the ion implantation treatment described in step b), a heat treatment is applied at a temperature approximately between 250° C and 800° C.

23. An endo-osseous implant or medical prosthesis, composed of:
a) A base material made from a metal alloy or metallic matrix composite.
b) An ion implantation, at least on the surface intended to be in contact with the bone, with at least one of the ions: C, O, H, N, CO, and/or a compound that comprises one or more of the said ions.
c) A microrugosity, at least on the surface intended to be in contact with the bone.
d) An induced oxide layer, at least on the surface intended to be in contact with the bone.

24. An endo-osseous implant or medical prosthesis, composed of:
a) A base material made from a metallic alloy or metallic matrix composite.
b) An ion implantation, at least at the surface intended to be in contact with the bone, with at least one of the ions: C, O, H, N, CO, and/or a compound that comprises one, or several of the said ions.
c) A microrugosity, at least on the surface intended to be in contact with the bone.

25. An endo-osseous implant or medical prosthesis, composed of:
a) A base material made from a metallic alloy or a metallic matrix composite.
b) An ion implantation, at least on the surface intended to be in contact with the bone, with at least one of these ions: C, O, H, N, CO, and/or a compound that comprises one or several of the said ions.
c) An induced oxide layer, at least on the surface intended to be in contact with the bone.

26. An endo-osseous implant or medical prosthesis, according to claims 23, 24 and 25, **characterized in that** the implant or the prosthesis is one of the following: dental implant, prosthesis for hip, knee, shoulder, elbow, wrist, finger or vertebra.

27. An endo-osseous implant or medical prosthesis according to claims 23 and 24 **characterized in that** the microrugosity produced, at least at the surface intended to be in contact with the bone, is produced by micro-shot-peening or shot-blasting.

28. An endo-osseous implant or medical prosthesis according to claims 23 and 24 **characterized in that** the microrugosity has a typical value between 0.5 and 10 µm Ra.

29. An endo-osseous implant or medical prosthesis according to claims 23 and 25, **characterized in that** the oxide layer produced, at least at the surface to make contact with the bone, is produced by chemical attack, anodizing, heat treatment, acidic attack at temperature or chemical conversion.

30. An endo-osseous implant or medical prosthesis according to claim 29, **characterized in that** the layer is more than 15 nanometres of thickness.

31. An endo-osseous implant or medical prosthesis according to claims 23, 24 and 25, **characterized in that** the ionic implantation, at least at the surface to make contact with the bone, produces a carbon-rich surface in which the composition of the oxide layer contains an average of more than 20% carbon in, at least, its first 20 nanometres of thickness.

32. An endo-osseous implant or medical prosthesis, according to claims 23, 24 and 25, **characterized in that** the ion implantation, in at least the surface intended to be in contact with the bone, produces a surface rich in carbon **characterized by** the presence of graphite bonds, titanium carbides rich in carbon and titanium carbides.

33. An endo-osseous implant or medical prosthesis according to claims 23, 24 and 25, **characterized in that** the ion implantation, at least on the surface intended to be in contact with the bone, produces a surface rich in carbon **characterized by** the presence of graphite bonds, titanium carbides rich in carbon, titanium carbides and CO bonds.

34. An endo-osseous implant or medical prosthesis according to claims 23, 24 and 25, **characterized in that** the ion implantation produces, at least on the surface intended to be in contact with the bone, a carbon-rich surface that is **characterized by** the presence of more than 10% graphite bonds along at least the first 10 nanometres of thickness.

35. An endo-osseous implant or medical prosthesis according to the 23rd, 24th and 25th claims, **characterized in that** the substrate material is obtained from titanium, titanium alloys, cobalt-chromium-based alloys, stainless steel or a metallic matrix composite with any of these alloys.

36. An endo-osseous implant or medical prosthesis according to claims 23, 24 and 25, **characterized in that** it presents a nanotextured surface.

37. An endo-osseous implant or medical prosthesis according to claim 36, **characterized in that** the nanotextured surface is approximately 3 to 6 nm Ra on a previously mirror-polished surface.

38. An endo-osseous implant or medical prosthesis according to claims, 23, 24 and 25 **characterized in that** it presents better hydrophilic properties than the base material.

39. An endo-osseous implant or medical prosthesis according to claims, 23, 24 and 25, **characterized in that** the ion implantation takes place in a residual atmosphere of oxygen, CO2 or some organic compound in the treatment chamber.

40. An endo-osseous implant or medical prosthesis according to claims 23, 24 and 25, **characterized in that** the ion implantation takes place during the evaporation of an organic compound in the treatment chamber.

41. An endo-osseous implant or medical prosthesis according to claims, 23, 24 and 25, **characterized in that** the ion implantation is applied by the "line of sight ion implantation" technique or "beam ion implantation", "plasma immersion ion implantation" or "plasma source ion implantation", or an ionic bombardment technique.

42. An endo-osseous implant or medical prosthesis according to claims, 23, 24 and 25, **characterized in that** the ion implantation is applied using an energy of 200eV to 1000keV, in a treatment chamber with a vacuum higher than 1 millibar and with an implanted dose of at least 10¹⁵ ions/cm².

43. An endo-osseous implant or medical prosthesis according to claims 23, 24 and 25, **characterized in that** after the ion implantation, a temperature between -120° C and 800° C is applied.

44. An endo-osseous implant or medical prosthesis according to claims 23, 24 and 25, **characterized in that** after the ion implantation a heat treatment is applied at a temperature of approximately 250° C to 800° C.
